# EUROPÄISCHE PATENTSCHRIFT

(11) **EP 1 774 981 B1**
(45) Veröffentlichungstag und Bekanntmachung des Hinweises auf die Patenterteilung: **20.08.2014**
(21) Anmeldenummer: 06023501.7
(22) Anmeldetag: 22.04.1999
(51) Int. Cl.: A61L 2/26

(54) **Modulares Sterilisierungsset**
Modular sterilising set
Set de sterilisation modulaire

(30) Priorität: 30.04.1998 DE 19819328
(43) Veröffentlichungstag der Anmeldung: 18.04.2007
(62) Teilanmeldung aus: 99927714.8
(73) Patentinhaber: Karl Storz GmbH & Co. KG, Postfach 230 78503 Tuttlingen (DE)
(72) Erfinder: Draenert, Klaus, 81545 München (DE)
(74) Vertreter: Vossius & Partner

(56) Entgegenhaltungen:
- WO-A-94/01055
- DE-A- 4 138 674
- DE-A- 19 611 680
- US-A- 4 643 303
- US-A- 5 061 018
- US-A- 5 540 901

## Beschreibung

Die vorliegende Erfindung betrifft ein Modulsystem aus reversibel anordnenbaren Haltern auf Lochblechen, die wiederum in modularer Bauweise so zusammengesetzt werden können, dass am Ort der Anwendung kürzestmögliche Wege und bestmögliche Übersicht über die Anordnung einzelner Instrumente erreicht werden kann. Dies ist dadurch notwendig geworden, da sich in der Vergangenheit vor allem bei chirurgischen Eingriffen gezeigt hat, dass einzelne wichtige Instrumente, die normalerweise vorverpackt in einem Sterilisationsset, welches für eine bestimmte Operation gerichtet worden war, fehlten, was aus dem Grunde nicht gleich erkannt worden war, da normalerweise die Instrumente zwar nach Checkliste, aber ungeordnet in sog. "Sieben" im Sterilisierbehälter sterilisiert werden und erst danach auf dem Tisch nach einer bestimmten Ordnung aufgebaut werden.

Normalerweise steht die Instrumentierschwester zwischen dem Instrumententisch auf der einen Seite und dem Operationstisch auf der anderen Seite.

Das Aufbauen der Instrumente erfolgt nach dem Herausnehmen aus den Sterilisationsboxen.

Das übersichtliche Anordnen wird nach einer bestimmten erfahrungsgemäss erarbeiteten Reihenfolge vorgenommen: Die Instrumente werden auf den Operationstischen, die wie eine Wagenburg um die Operationsschwester aufgebaut sind, angeordnet und meist über den Operationsflur dem Chirurgen an den Operationstisch angereicht. Das Aufbauen der Tische und das übersichtliche Anordnen der Instrumente benötigt meist eine Vorlaufzeit von mindestens einer halben Stunde. Passiert es nun, dass beim Herüberreichen eines wichtigen Instrumentes, welches auch noch eine gewisse Schlüpfrigkeit hat, beispielsweise eines Knochenbohrers, dieser der Schwester entgleitet und auf die unsterile Unterlage fällt, so verlien der Chirurg heute bis zu einer Stunde Zeit. In der Vergangenheit wurden bei solchen Zwischenfällen die Instrumente gereinigt und blitzsterilisiert, was aufgrund der Unsicherheiten der kurzen Sterilisiervorgänge nunmehr nicht mehr erlaubt ist. Die sorgfattige Validierung einzelner Operationen hat gezeigt, dass sowohl vom Ablauf der Operation her als auch von der Ergonomie der Instrumentierwege die Arbeiten, die ausschliesslich von der Schwester verrichtet werden, besser konzentriert sind in unmittelbarer Nähe der Operationsschwester, während alle die Instrumente, die auch vom Chirurgen benutzt werden, besser in unmittelbarer Nähe des Chirurgen angeordnet sind, so dass er ggf. auch durch direkten Zugriff sich des Instrumentes bedienen und dieses auch geordnet wieder an seinen vorherigen Platz zurücklegen kann. Für eine solche subtile, nach ergonomischen Gesichtspunkten erarbeitete Anordnung von Instrumenten und Geräten, einschliesslich der Energieversorgung (in der Regel Druckluft) gab es bisher keine zufriedenstellende Vorrichtung, die es erlaubt hätte, in modularer Weise die notwendigen Instrumente, in optimierter Form anzuordnen. Hinzu kommt, dass die relativ aufwendige, optimierte Anordnung von Instrumenten und Geräten nicht in unmittelbaren Zusammenhang mit der Operation, bei der sowohl die Operationsschwester als auch der Chirurg von anderen Prioritäten besetzt sind, optimal durchgeführt werden kann, sondern vielmehr besser am Tag zuvor nach dem Reinigen der Instrumente vorgenommen werden sollte. Im modularen Sterilisationsset angeordnet, werden die Instrumente gleich in fertiger Anordnung sterilisiert und in sog. "modulen Stecksystemen" unmittelbar am Operationstisch auf einem speziellen Instrumentiertisch individuell angeordnet. Die gesamte Zeit des Aufbaus verkürzt sich dadurch auf etwa 5 - 10 Minuten. Das Problem konnte in sehr einfacher Weise durch solche fein abgestimmten Modulsysteme gelöst werden, die der folgenden Erfindung zugrunde liegen.

Der Stand der Forschung und der angewandten Praxis in den instrumentierzentren, wie Operationssälen oder ambulanten Praxen, ist der, dass die Instrumente für eine Operation auf freien nicht strukturierten Operationstischen, welche mit Operationstüchem abgedeckt werden, aufgebaut werden, zum Teil einzeln oder in einer Serie von gleichen Instrumenten direkt auf den Tisch oder in einzelnen geöffneten und steril abgedeckten Sterilisationsboxen, die einzeln oder nebeneinander aufgebaut werden und die darin enthaltenen Instrumente einsehen lassen.
Aus der US 4,643,303 ist eine Vorrichtung zum Sterilisieren, Transportieren und Lagern einer Vielzahl von chirurgischen Instrumenten bekannt, die einen Drahtkorb umfasst, der von einem schachtelförmigen, mit einem Deckel verschliessbaren Behältnis aufgenommen werden kann. Im Drahtkorb lassen sich eine Mehrzahl von Instrumenten-Träger-Modulen anordnen, die derart geformt sind, dass sie jeweils eine Mehrzahl von identischen Instrumenten halten können. Die Träger-Module weisen eine im wesentlichen rechteckige Grundfläche auf und können variabel im ebenfalls rechteckigen Drahtkorb angeordnet werden. Die Träger-Module weisen derart ausgebildete Instrumenten-Aufnahmeaufnehmungen auf, die es erlauben die Instrumente zur Vorbereitung einer Operation aus einer platzsparenden Sterilisierstellung in eine entsprechende Entnahmestellung im Trägermodul anzuordnen.
In der WO94/01055 ist ein modulares Kassettensystem zur Sterilisation von zahnmedizinischen Instrumenten beschrieben. Eine Bodenplatte und ein Deckel sind an den einander zugewandten Seiten mit einem dichten Raster von hohlzylindrischen Nocken versehen. Im Nockenraster an Basis und Deckel lassen sich verschiedene Instrumentenhalter platzieren, die bei geschlossener Kassette derart zusammenwirken, dass verschiedene dentalchirurgische Instrumente fest zwischen den oberen und unteren Haltern klemmend gehalten sind.
Aus US 5,540,901 A ist ein modulares Sterilisationssystem für medizinische Instrumente bekannt mit einem im Wesentlichen rechteckigen Behälter, der ein Paar gegenüberliegender Seitenwände, ein Paar gegenüberliegender Endwände und eine Bodenwand aufweist. In den Seiten- und Endwänden sind mehrere vertikale Säulen von vertikal beabstandeten Löchern gleichmäßiger Größe vorgesehen. Zur Anordnung im Behälter sind ein oder mehrere im Wesentlichen rechteckige Instrumentenablagen vorgesehen, wobei die Länge und Breite jeder Ablage direkt mit dem Abstand der Säulen der Löcher in den Seiten- und Endwänden des Behälters verknüpft sind, so dass ein oder mehrere Ablagen innerhalb des Behälters positioniert werden können, so dass sie von ausgewählten Säulen von Löchern eingeklammert werden.

Vereinzelt gibt es weiter strukturierte sog. "SteriLisationssiebe", die einzeln unterteilt sind, so dass einzelne Fächer entstehen, in denen bestimmte Instrumente oder Implantate übersichtlich angeordnet abgelegt werden können. Vereinzelt, z. B. im Instrumentenkatalog der Firma Martin Medizin-Technik in Tuttlingen, Ausgabe 8/97, gibt es für die aus Lochblechen gefertigten Sterilisationssiebe Metallstifte, sogenannte "Sortierstifte" mit Artikelnummer 55 991 09 MA, die an einem Ende eingefräst sind, so dass eine komprimierbare Metallspange entsteht, die man zusammendrücken kann; in diesem Zustand lassen sich die Stifte in die Lochbleche einstecken. Bei einem solchen Stift lassen sich einzelne Instrumente auffädeln; durch die Anwendung von drei Stiften, in entsprechender Anordnung lassen sich auch einzelne gerade oder leicht gekrümmte Instrumente oder Implantate lose in einer bestimmtenAnordnung plazieren. Keinesfalls ist es aber möglich, mit diesem System in übersichtlicher Weise verschiedene Instrumente in fester Anordnung unterzubringen, schon gar nicht die Vielfalt von Instrumenten, die für eine Operation benötigt werden. Darüber hinaus wird die Anwendung der Stifte dadurch erschwert, dass sie sich kaum oder nur schwer im Blech verankern lassen.

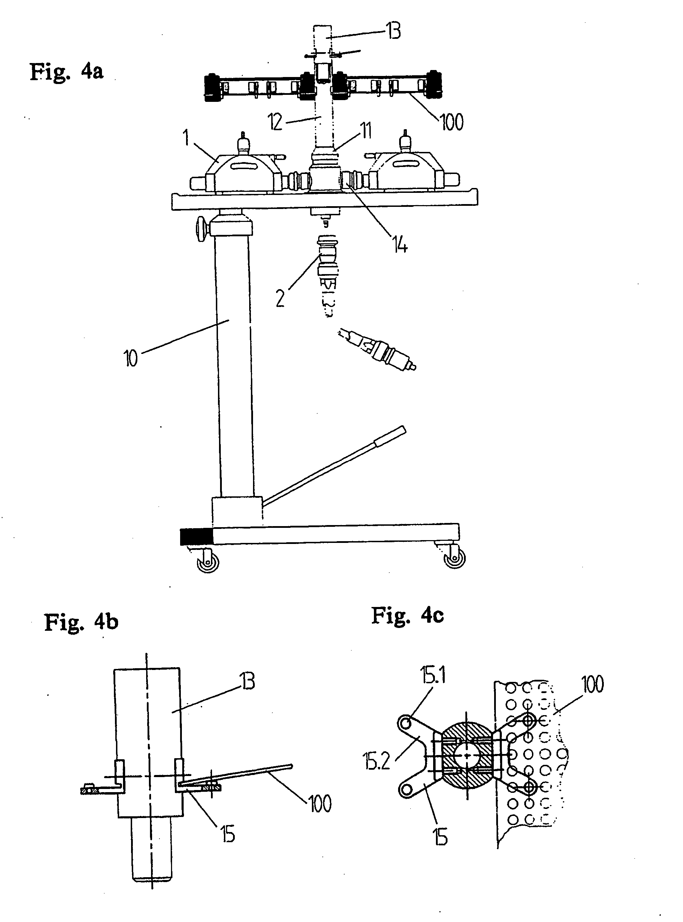

Keines der vorhandenen Systeme ist in der Weise stapelbar, dass Instrumente in fester und vorgegebener Anordnung miteinander verbunden werden können, so dass ein geschlossenes Herausnehmen aus dem Sterilisationsgefäss möglich ist. Auf diese Weise wird ein Instrumentierweg beim Richten des Instrumentierarbeitsplatzes gespart. Die Erfindung ist durch die Merkmale der Patentansprüche definiert. Am einfachsten zu verstehen ist die Erfindung, die alle diese Probleme sehr einfach und elegant löst, beim Durchführen einer Hüftgelenkersatiogeration: beispielsweise benötigt man Geräte zur Erzeugung des Vakuums in unmittelbarer Nähe des Operationstisches; man benötigt Bohrmaschinen und Geräte zum Anmischen von Knochenzement und zum Applizieren von Knochenzement; daneben benötigt man Implantate und eine Reihe von Instrumenten, die zum Teil spezifischer, zum Teil allgemein chirurgischer Art sind. Das Aufbauen eines solchen Instrumentariums in Form der geschilderten Wagenburg um die Operationsschwester herum dauert mindestens 30 bis 40 Minuten. Wird nun eine solche Operation mit dem sog. "Modulsystem" vorbereitet, so sind bereits am Tag vor der Operation alle Instrumente in fester Position in reversibler Befestigung, aber für diese Operation fest angeordneten Weise, im Sterilisationsset untergebracht und in diesem sterilisiert. Sie können am Tag der Operation geordnet in dieser Weise herausgenommen und an einer modularen Säule befestigt werden, welche ebenfalls sterilisert werden kann, und in der Weise angeordnet ist, dass sie in einem Basismodul verankert wird, aus der gleichzeitig die Druckluftversorgung als Energieversorgung für die Betriebsmaschinen zum Bohren, Fräsen und Zementapplizieren genommen werden kann. Ein solcher Operationstisch ist in der Fig. 4a dargestellt. Er besteht aus einem normalen U-förmigen Operationstisch mit einer zentralen Aufnahme für eine zentrale Säule (Fig. 4a). Das Tablett des Instrumententisches wird normal mit einem sterilen Sack steril abgedeckt. Dieser wird mit der zentralen Säule perforiert und das sterile und für Flüssigkeiten undurchdringbare Abdecktuch wird so zwischen Säule und Tisch eingeklemmt, dass keine Kolonisierung von sterilen Oberflächen mit Bakterien erfolgen kann. Der Operntionstisch kann in derseiben Weise auch durch einen doppelten Disposable-Abdecksack abgedeckt werden. Die Säule nimmt nicht nur die Antriebsmaschinen auf, sondern kann im Zentrum kleine und grössere Module aufnehmen, an denen wiederum fertig gepackte Instrumentenseis, welche aus den Sterilboxen entnommen werden, angedockt sind und in übersichtlicher Weise angeordnet werden. In dieser Weise kann individuell für jede Hüftgelenkersatzoperation ein Set mit Implantaten von Pfannen und Femoralkomponenten und darüber ein oder zwei Sets mit spezifischen Instrumenten angeordnet werden. Das Besondere an diesem Modularsystem ist, dass jedes einzelne Instrument dadurch, dass eine Art Baukastensystem von Haltern mit Aufnahmen für die Instrumente besteht, fest angeordnet werden kann. Wird ein Instrument für die Operation gerichtet, so ist es entsprechend der Reihenfolge der Anwendung in übersichtlicher Weise fest auf dem Lochbrett fixiert, gegebenenfall s beschriftet und zwischen Haltern fest gelagert, aus denen es herausgenommen werden kann. Ist dieses Instrument einmal benützt, so kann es beispielsweise auf einen der beiden Halter, die für die feste Anordnung des Instrumentes notwendig sind, in aufrechter Position aufgesteckt werden. Der Chirurg weiss sofort, was das zuletzt benutzte Instrument war und kann dementsprechend entweder dieses oder das im Operationschritt nächstfolgende Instrument einsetzen.

Wird während der Operation von der Schwester etwas vorbereitet, wie beispielsweise das Vakuummischen von Knochenzement, so kann der Chirurg dennoch ohne Zeitverlust weiter operieren, kann sein Operationsgebiet für den Eingriff, für den die Schwester den Knochenzement vorbereitet, sorgfältig präparieren. Die Schwester kann sich auf ihre Tätigkeit konzentrieren und der Chirurg ist für diesen Moment unabhängig von der Instrumentierschwester und kann die einzelnen Instrumente sich selbst greifen und in derselben Weise auch selbst zurücklegen. Die Anordnung der Instrumente ist am Tag vor der Operation bereits festgelegt worden, in dem die Instrumente für den Chirurgen ihm nah und die, die von der Schwester gebraucht werden, auf der Seite der Schwester angeordnet sind. Alle Versorgungsleilungen sind kurz und zwar zwischen der zentralen Säule und dem Operationstisch bzw. zwischen der Einheit der Schwester und der zentralen Säule. Meist kommen diese Versorgungseinheiten ohne Schlauchsystem aus und sind direkt angeflanscht (Fig. 4a). Die einzelnen Instrumentensets sind einfach auf die jeweiligen Module aufsteckbar. Die Länge der Module wird jeweils so gewählt, dass sie frei drehbar sind über den auf der Basisplatte des Instrumentiertisches angeordneten Geräten (Fig. 4b). Die Halter sind ohne irgendwelche Schraubverbindungen, so dass sie schnell eingehängt und eingesteckt werden können (Fig. 4c). Die am Vortag gerichteten Instrumente und am selben Nachmittag nach der Operation wieder hergerichteten Instrumente sind, nachdem sie gereinigt und von Rückständen befreit worden sind, übersichtlich und in der Reihenfolge, in der sie benützt werden, auf dem Lochbrett in fester Position angeordnet und können aus diesen Haltern sehr leicht herausgenommen und nach Gebrauch auch in anderer Position aufrecht aufgesteckt werden. Auf diese Weise ist es in sehr einfacher Anordnung möglich, ein sehr wichtiges Instrument, welches gerne zu Boden fällt, in doppelter Ausgabe übersichtlich auf dem Instrumentierblech zu positionieren.

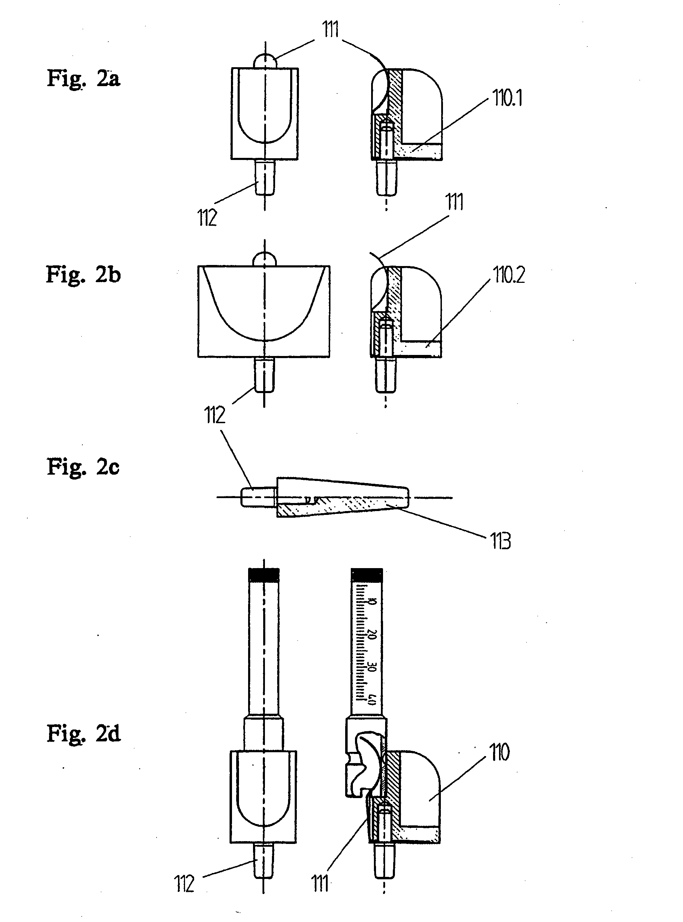

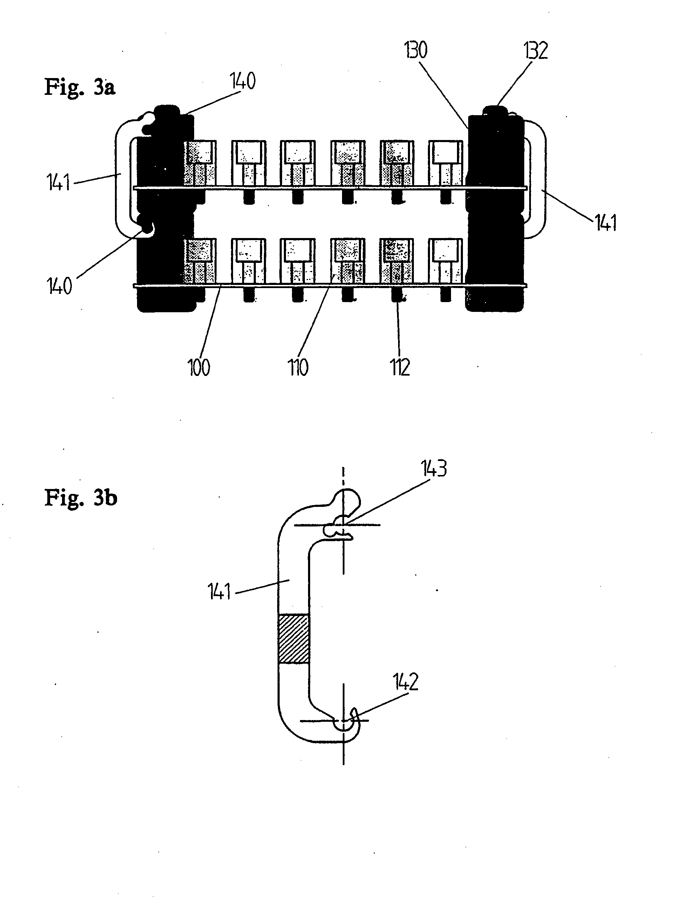

Das Risiko einer Operation wird umso kleiner, je sorgfältiger die Planung eines solchen Sets vorgenommen wurde und je vollständiger ein Packen des Sets validiert wurde, d.h. entsprechend einer Checkliste am Tag vor der Operation hingerichtet wurde. Die einzelnen modularen Halter für die Instrumente sind so beschaffen (Fig. 2a - d), dass jedes Instrument mit einem, meistens zwei Haltern fest fixiert werden kann. Instrumentensets, die zusammengehören und nacheinander benützt werden, oder auch nur um diese zusammen aus der Sterilbox zu entnehmen, da sie übereinander auf der zentralen Säule angeordnet werden, können auf sehr einfache Weise über die Griffleisten fest aneinander gekoppelt werden, so dass sie zusammen aus der Sterilbox entnommen werden können (Fig 3a und 3b). Ein besonderer Vorteil dieses modularen Systems ist es, dass es für jede Operation in freier Kombination so optimiert werden kann, dass aufgrund der verkürzten Instrumentierwege und aufgrund der vorgegebenen und vorgepackten Anordnung die Zeiten im Operationssaal zwischen 20 Minuten bis zu einer halben Stunde gekürzt werden können. Die Instrumentienung, dies hat sich gezeigt (Europilotstudie des Zentrums für Orthopädische Wissenschaften, München ,1995 - 1998) war einer der wichtigten Faktoren für die Verkürzung der Operationszeiten und für die Sicherheit des operativen Eingriffes, da jedes Improvisieren des Chirurgen aufgrund des Fehlens eines Instrumenten wegfällt.

Die Erfindung wird nachstehend anhand der Figuren näher erläutert.

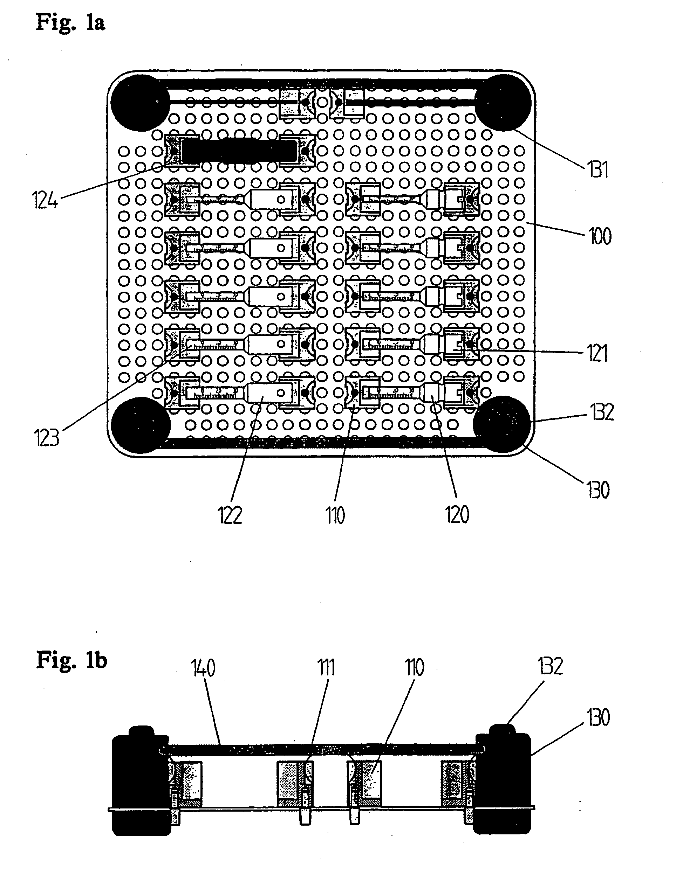

In den Figuren 1a und 1b ist ein kleines Transplantationsset, bestehend aus Diamantfräsen 120 und den sogenannten Extraktoren 122 dargestellt. Mit einem Paar von diamantbeschichteten Fräsen 120 und 121 können freie Knorpel-Knochen-Transplantate durchgeführt werden, Ein solches Zwillingsinstrumentarium ist auf 1/10 mm aufeinander abgestimmt und das Verwechseln der äusserlich sehr ähnlichen Instrumente würde das Operationsergebnis zunichte machen. Die Tatsache, das die Instrumente in fester Reihenfolge angeordnet sind und die Tatsache, dass ein gebrauchtes Instrument am Ende des Halters in vertikaler Position aufgesteckt werden kann (Fig. 2a), vermeidet diesen Fehler, da jedes Transplantat , wenn es mit dem Diamant geschliffen worden ist, mit einem dazu direkt korrelierenden Instrument, dem sogenannten Extraktor 122 herausgenommen wird und nur das Instrumentenpaar 120 / 122 aufeinander abgestimmt ist, resp. das Instrumentenpaar 121 /123 u.s.w. Ein Verwechseln dieser Zuordnung würde zu dramatischen Komplikationen führen, indem Transplantat oder Trünsplantatbett zerstört würden. Der für die Extraktion notwendige Handgriff (124) ist übersichtlich in der passenden Grösse den Extraktoren zugeordnet für die dieser entsprechende Handgriff 124 passt.

Durch entsprechende Anordnung von Eckmodulen 130, die in sich wiederum Aufnahmen 131 für Instrumente beinhalten können, wird erreicht, dass die einzelnen Sterisets übereinander gestapelt werden können und in einfacher Weise an einer Griffleiste 140; auch in heissem Zustand; aus der Steribox herausgenommen werden können.

In der Fig. 3a ist dargestellt, dass zwei solcher Griffleisten sehr leicht und sicher durch mindestens zwei Klammerhaltter 141, welche in Schnappverschlüssen 140 einrasten, aneinandergekoppelt werden können. Die Klammerbalter (Fig. 3b) sind so konstruiert, dass sie in der unteren Griffleiste eingehakt und in der oberen Griffleiste eingeschnappt werden, wobei ein kleiner Widerstand überwunden werden muss (142/ 143 der Fig. 3b). Die sorgfältige Beobachtung von Instrumentierabläufen und Validierung der einzulnen Schritte liess erkennen, dass es sehr hilfreich ist, wenn das eben benutzte Instrument in anderer Position zurückgesteckt werden kann, was sehr einfach durch die Erfindung, welche in der Fig. 2a bei 111 dargestellt ist, möglich ist. Auf diese Weise erkennt der Chirurg die Diamantfräse; mit der er das Transplantat entnommen hat, und kann so für die Vorbereitung des Transplanlatbettes die nachfolgende Fräse (121 in Fig. 1a) wählen Erst wenn die heikle Phase der Operation, die Abstimmung von Transplantatbett und Transplantat erfolgreich beendet wurde, werden die Instrumente wieder in ihre Halter zurückgelegt. Besondere Instrumente, wie Scheren und andere allgemeine Instrumente werdenvorteilhafterweise von Haltem aufgenommen, die in Fig. 2c dargestellt sind. Auch Prothesen lassen sich vorteilhaft zwischen Haltern der Art wie in Fig. 2a, bei 110.1 und Fig. 2b bei 110.2, respektive Fig. 2c bei 113, auch einzeln oder in Serie sicher positionieren.
Die Vorrichtung ist gemäss der Erfindung so beschaffen, dass die Halter mindestens eine konkave, meist nach oben offene Aufnahme verschiedener Weite und Tiefe vorweisen, die eine sichere Aufnahme von Instrumenten ermöglicht.
Eine Ausführungsform der erfindungsgemässen Vorrichtung ist so beschaffen, dass die Halter Konen mit verschiedenen Durchmessern und Steigungen darstellen, die Instrumente mit Scherengriffen und Fingerhaltern aufnehmen können.
Gemäss einer weiteren Ausführungsform ist die Vorrichtung so beschaffen, dass Halter zumindest paarweise verschiedene Farben haben und/oder Markierungen tragen.
Ein fertig eingerichtetes In-strumentenset wird notwendigerweise direkt in unmittelbarer Nähe des Operationsfeldes benötigt. In der Anordnung auf einem solchen Operationstison 10 in Fig. 4a-c ist über die zentrale Säule 12, 13 das Instrumentierset 100 frei drehbar über dem Gerät 1, sowohl für die Seite des Chirurgen, als auch für die Seite der Instrumentierschwester erreichbar.

## Patentansprüche

1. Vorrichtung zum Sterilisieren und Bereitstellen von chirurgischen Instrumenten, welche, wenn sie sterilisiert sind, in vorgegebener Anordnung benutzbar sind, mit einer Basisplatte (100) mit Löchern für die Passage eines Sterilisiermediums, und mindestens zwei Haltern (110), welche aus einem Teil (112) zum reversiblen Befestigen in den Löchern der Basisplatte (100) und einem zentralen Körper (110.1, 110.2, 113) bestehen, wobei die Halter so beschaffen sind in Größe und Form, dass sie einzeln (110.1, 110.2, 113) und/oder in Kombination mit mindestens einem weiteren Halter (110) zumindest ein Instrument fest in sich aufnehmen können, **dadurch gekennzeichnet, dass** die Halter (110) mindestens eine konkave, nach oben offene Aufnahme verschiedener Weite und Tiefe aufweisen und dass einzelne Halter (110) so beschaffen sind, dass sie vor ihrer operativen Anordnung die Instrumente tragen und nach Gebrauch dieselben in anderer Position (111) aufnehmen können.

2. Vorrichtung nach Anspruch 1, wobei die Halter Konen mit verschiedenen Durchmessern und Steigungen darstellen, die Instrumente mit Scherengriffen und Fingerhaltern aufnehmen können.

3. Vorrichtung nach Anspruch 1 oder 2, wobei die Lochplatte (100) an allen Ecken hochragende Eckmodule (130) aufweist, die so beschaffen sind (132), dass ein oder mehrere Platten übereinander gestapelt und miteinander verbunden werden können.

4. Vorrichtung nach den Ansprüchen 1 bis 3, wobei an mindestens zwei Enden der Basisplatte (100) Griffe (140) montiert sind, so beschaffen, dass mindestens zwei Lochplatten (100) übereinander gestapelt, beispielsweise durch Schnappklammem (141), miteinander verbunden werden können.

5. Vorrichtung nach den Ansprüchen 1 bis 4, so beschaffen, dass die Halter (110) zumindest paarweise verschiedene Farben haben und/oder Markierungen tragen.

6. Vorrichtung nach den Ansprüchen 1 bis 5, so beschaffen, dass der Befestigungsteil (112) zur reversiblen Fixation in der Basisplatte (100) ein konischer Stift ist, der sich press-fit in der Platte (100) festsetzt.

7. Vorrichtung nach den Ansprüchen 1 bis 6, so beschaffen, dass mindestens eine Basisplatte (100) an einer zentralen Säule (12, 13) befestigt werden kann mit einem Halter für ein Lochblech (15), der mit einem Teil an einer Säule (12, 13) verankert wird und eine Aufnahme (15.2) für das Lochblech (15) aufweist und mit einem anderen Teil (15.1) reversibel in der Basisplatte (100) verankert werden kann.

8. Vorrichtung nach den Ansprüchen 1 bis 7, so beschaffen, dass sie reversibel an einer Säule befestigt werden kann, die als Modul ausgebildet ist, bestehend aus einem Körper als Säule (12, 13) zur Aufnahme von mindestens einem Halter für ein Lochblech (15) und einer Aufnahme für ein weiteres Modul in seinem Kopfteil (13) und einem Fußteil, mit welchem das Modul in einer zentralen Basissäule (11) verankert werden kann.

9. Vorrichtung nach Anspruch 8, wobei die zentrale Basissäule mindestens eine Versorgungsleitung für Druckluft mit Abluftkanälen enthält und aus einem Fußteil und einem Körper besteht, dessen Fußteil nach der Sterilisation durch eine sterile Abdeckung hindurch reversibel in einer Aufnahme in einem Instrumententisch (10) verankert werden kann und ein Schnellkupplungsteil für die Druckluftzufuhr aufweist und dessen Körper mindestens eine Schnellkupplung (2) für das Anschließen eines Druckluftschlauches beinhaltet.

## Claims

1. A device for sterilizing and providing surgical instruments which are, when sterilized, usable in a predetermined arrangement having a base plate (100) with holes for the passage of the sterilization medium, and at least two holders (110), which consists of a part (112) for the reversible fastening in the holes of the base plate (100) and of a central body (110.1, 110.2, 113), designed with regard to size and shape such that individually (110.1, 110.2, 113) and/or in combination with at least one further holder (110), they may firmly receive at least one instrument, **characterized in that** the holders (110) comprise at least one concave receiver, which is open to the top, of a different width and depth and that individual holders (110) are designed such that they carry the instruments before their operative arrangement and after use receive these in a different position (111).

2. The device of claim 1 wherein the holders represent cones with different diameters and gradients, which may receive instruments with scissor grips and finger holders.

3. The device of claim 1 or 2, wherein the perforated plate (100) comprises angle modules (130) projecting upwardly at all angles which are designed such (132) that one or more plates can be stacked on top of one another and connected to each other.

4. The device of claims 1 to 3 wherein handles (140) are arranged at at least two ends of the base plate (100), designed such that at least two perforated plates (100) can be stacked on top of one another and connected to each other, for example by means of snap-fit clips (141).

5. The device of claims 1 to 4 designed such that the holders (110) at least in pairs have different colours and/or carry markings.

6. The device of claims 1 to 5 designed such that the fastening part (112) for the reversible fixation in the base plate (100) is a conical pin which locks in the plate (100) with a press-fit.

7. The device of claims 1 to 6, designed such that at least one base plate (100) may be fastened to a central column (12, 13) via a holder for a perforated plate (15) anchored with a part on a column (12, 13), and which comprises a receiver (15.2) for the perforated plate (15) and may be anchored reversibly with another part (15.1) in the base plate (100).

8. The device of claims 1 to 7, designed such that it may be reversibly fastened on a column which is designed as a module consisting of a body as column (12, 13) for receiving at least one holder for a perforated plate (15) and of a receiver for a further module in its head part (13) and of a foot part with which the module may be anchored in a central base column (11).

9. The device of claim 8 wherein the central base column contains at least one supply conduit for pressurized air with discharge air channels, and consists of a foot part and a body, whose foot part after sterilization may be reversibly anchored through a sterile covering, in a receiver in an instrument table (10) and comprises a quick coupling part for the supply of pressurized air and whose body contains at least one quick coupling (2) for the connection of pressurized air flexible tubing.

## Revendications

1. Dispositif pour la stérilisation et la mise à disposition d'instruments chirurgicaux, lesquels sont utilisables suivant un agencement défini une fois stérilisés, comportant une plaque de base (100) avec des trous pour le passage d'un fluide de stérilisation, et au moins deux supports (110) qui sont constitués d'une partie (112) pour la fixation réversible dans les trous de la plaque de base (100) et d'un corps central (110.1, 110.2, 113), lesdits supports présentant une taille et une forme telles qu'ils peuvent recevoir fixement en eux au moins un instrument, individuellement (110.1, 110.2, 113) et/ou en combinaison avec au moins un autre support (110), **caractérisé en ce que** les supports (110) présentent au moins un logement concave, ouvert vers le haut, de différentes largeurs et profondeurs, et **en ce que** les différents supports (110) sont configurés de telle sorte qu'ils peuvent supporter les instruments avant leur agencement opératoire, et recevoir ceux-ci dans une autre position (111) après utilisation.

2. Dispositif selon la revendication 1, où les supports de soutien figurent des cônes de diamètres et de conicités différents, pouvant recevoir des instruments avec des anneaux de ciseaux et des prises pour les doigts.

3. Dispositif selon la revendication 1 ou la revendication 2, où la plaque perforée (100) présente des modules de coin (130) dressés dans tous ses coins, configurés (132) de manière à permettre l'empilement par superposition et l'assemblage d'une ou de plusieurs plaques.

4. Dispositif selon les revendications 1 à 3, où des poignées (140) sont montées sur au moins deux extrémités de la plaque de base (100), configurées de manière à permettre l'empilement et l'assemblage d'au moins deux plaques perforées (100), notamment au moyen de brides enclenchables (141).

5. Dispositif selon les revendications 1 à 4, configuré de telle sorte que les supports (110) présentent au moins des couleurs et/ou des marquages différents par paires.

6. Dispositif selon les revendications 1 à 5, configuré de telle sorte que la partie de fixation (112) pour la fixation réversible dans la plaque de base (100) est une goupille conique qui se fixe par sertissage dans la plaque (100).

7. Dispositif selon les revendications 1 à 6, configuré de telle sorte qu'au moins une plaque de base (100) peut être fixée sur une colonne centrale (12, 13) avec un support pour une tôle perforée (15), lequel est ancré par une partie sur une colonne (12, 13) et présente un logement (15.2) pour la plaque perforée (15), et peut être ancré de façon réversible dans la plaque de base (100) par une autre partie (15.1).

8. Dispositif selon les revendications 1 à 7, configuré de telle sorte qu'il peut être fixé de façon réversible à une colonne réalisée comme un module composé d'un corps en tant que colonne (12, 13) pour la réception d'au moins un support pour une tôle perforée (15) et d'un logement pour un autre module dans sa partie de tête (13), et d'une partie inférieure par laquelle le module peut être ancré dans une colonne de base centrale (11).

9. Dispositif selon la revendication 8, où la colonne de base centrale contient au moins une conduite d'alimentation en air comprimé avec des canaux d'évacuation d'air, et est constituée d'une partie inférieure et d'un corps, dont la partie inférieure peut après stérilisation être ancrée de façon réversible dans un logement d'une table d'instruments (10) au travers d'un couvercle stérile et présente une pièce de raccord rapide pour l'amenée d'air comprimé, et dont le corps comprend au moins un raccord rapide (2) pour la connexion d'un tuyau à air comprimé.
